# EUROPEAN PATENT SPECIFICATION

(11) **EP 1 121 928 B1**
(45) Date of publication and mention of the grant of the patent: **23.01.2008**
(21) Application number: 01300793.5
(22) Date of filing: 30.01.2001
(51) Int. Cl.: A61K 31/045, A61K 31/575, A61P 9/00

(54) **Compositions containing phytosterol and policosanol esters of fatty acids for reducing blood cholesterol and triglycerides**
Zusammensetzungen enthaltend Phytosterol- und Policosanolester von Fettsäuren zur Herabsetzung des Cholesterol- und Triglyceridspiegels
Compositions contenant des ester d'acides gras de phytostérol et policosanol pour réduire le niveau de cholésterol et des triglycérides

(30) Priority: 31.01.2000 CL 2000209
(43) Date of publication of application: 08.08.2001
(73) Proprietor: Härting S.A., Santiago (CL)
(72) Inventor: Schersl, Endre Markovits, Quilpué (CL)
(74) Representative: Crouch, David John

(56) References cited:
- WO-A-00/61771
- WO-A-95/16434
- WO-A-98/06405
- WO-A-99/40922
- US-A- 5 958 913
- PATENT ABSTRACTS OF JAPAN vol. 012, no. 092 (C-483), 25 March 1988 (1988-03-25) & JP 62 224258 A (JUN KAWAI), 2 October 1987 (1987-10-02)
- PATENT ABSTRACTS OF JAPAN vol. 011, no. 315 (C-451), 14 October 1987 (1987-10-14) & JP 62 099323 A (YOSHIHIDE HAGIWARA), 8 May 1987 (1987-05-08)

## Description

The present invention is related to food and pharmaceutical compositions and methods suitable for lowering cholesterol and triglycerides level or elevating HDL-cholesterol level in blood of a mammal, particularly compositions containing esters of phytosterols and omega-3 or omega-6 polyunsaturated fatty acids and policosanols esters.

### Background

Disorders of lipid metabolism, especially the harmful effects caused by high cholesterol and triglycerides level in blood , have been intensively studied for many decades.

Cholesterol level in blood over 200 mg/dl constitutes the main risk factor of coronary diseases, the most frequent cause of death, principally in developed countries. However, the risk factor is not only related to high cholesterol level in blood, but also to the different forms of total cholesterol. A high level of low-density lipoprotein or LDL-cholesterol and very low-density lipoprotein or VLDL-cholesterol in blood constitutes a problem because these lipoproteins are very likely to remain in the cardiovascular system causing the formation of plaques in the coronary arteries. Likewise, low levels of high-density lipoproteins or HDL-cholesterol constitute an additional risk factor because they are useful in removing the form of cholesterol that blocks arteries. Therefore total cholesterol level and total cholesterol/HDL-cholesterol ratio must be considered for evaluating the risk of coronary diseases.

However, not only cholesterol, but also high triglycerides level in blood constitutes a risk factor of coronary diseases and other complications *(*PUFA NEWSLETTER, vol.2, June 1998).

In general, the treatment of lipid metabolism disorders has been mostly addressed to treating hypercholesterolemia using different food and pharmaceutical compositions that lower elevated cholesterol level in blood. Many of these compositions contain plant sterols or phytosterols which would interfere or obstruct the intestinal absorption of dietary cholesterol and reduce LDL-cholesterol. There is a vast scientific production related to this subject which is reviewed in U.S. Patent 5,958,913 quoting over 70 references concerning the effects and mechanisms of dietary phytosterols on the reduction of blood cholesterol.

U.S. Patent 5,244,887 discloses a method for the elaboration of a composition to be used as a food additive which contains one or more stanols, a solubilizing agent, an antioxidant and a dispersing agent. The stanols are obtained by catalytic hydrogenation of sterols. These food compositions are intended for reducing cholesterol absorption from foods.

U.S. Patent US 5,932,652 discloses a water dispersive food composition for reducing cholesterol absorption containing sitostanol (beta-sitostanol) and lecithin.

In order to increase the inhibition of dietary cholesterol absorption U.S. Patent 5,591,836 discloses a method that uses a saponin compound containing 5-C-hydroxymethylhexose and sterol or terpene.

U.S. Patent 5,747,464 discloses the utilization of complexes formed by beta-sitostanol and pectin. Sterols esterified with fatty acids seem to be more efficient cholesterol absorption inhibitors than free sterols.

U.S. Patent 5,958,913 discloses the utilization of stanol esters, mainly the fatty acid ester of beta-sitostanol, where the fatty acids are derived from raps seed oil. This patent also presents long clinical studies on the efficiency of these esters for inhibiting intestinal absorption of cholesterol and the lowering LDL-cholesterol in blood.

Long chain lineal saturated primary alcohols from 20 to 38 carbon atoms also called fatty alcohols or higher aliphatic alcohols also known as policosanols are efficient to reduce blood cholesterol.

In the present invention the term policosanol is used as meaning a lineal saturated primary alcohol containing 20 or more carbon atoms. The mechanism of action of policosanols is not known with certainity, but it is believed they would affect synthesis of cholesterol in the liver. A considerable reduction of total cholesterol level and LDL-cholesterol level in the blood of patients with diabetes mellitus upon sustained ingestion of small amounts of policosanols have been observed (Omayda Torres et al., Diabetes Care, "Treatment of Hypercholesterolemia in NIDDM with Polycosanol", 1995, vol. 18, N°5, 393-396).

U.S. Patent 5,856,316 discloses a process for obtaining policosanols from sugarcane wax and their utilization in the treatment of hypercholesterolemia. Policosanols from sugarcane wax comprise a mixture of aliphatic alcohols from 24 to 34 carbon atoms and they were effective hypocholesterolemic agents administered in daily doses from 1 to 100 mg.

U.S. Patent 5,952,893 discloses a composition for reducing cholesterol level in blood comprising a mixture of phytosterols (mixture of different plant sterols) and policosanols with a synergistic effect. Phytosterols of the invention comprise beta-sitosterol, campesterol and stigmasterol derived from vegetable oils and the policosanols of the invention comprise a mixture of fatty alcohols containing from 22 to 36 carbon atoms derived from rice bran wax. These policosanols are commercially available ("Rice Brad Wax", Traco Labs Inc.). However, free phytosterols and free policosanols are barely soluble in the micelle phase of food channels, therefore its efficiency to reduce blood cholesterol is rather low, which leads to the necessity of using relatively high doses of these compounds.

In addition, food and pharmaceutical compositions containing free phytosterols and/or free policosanols are not effective for reducing triglycerides level in blood.

The present invention is directed to a composition as set out in any one of the accompanying claims 1 and 5 Sub-claims 2 to 4 and 6 to 10 inclusive, set out preferred features.

Accordingly, the present invention may provide food and pharmaceutical compositions for lowering LDL-cholesterol level or elevating HDL-cholesterol level, or both, in the blood of a mammal, said compositions containing easily absorbable forms of policosanols in the digestive tract of said mammal, said easily absorbable forms of policosanols comprising an ester of a policosanol and a carboxilic acid preferably containing from 2 to 22 carbon atoms, denoted simply as a policosanol ester.

The present invention enables a method for lowering LDL-cholesterol or elevating HDT,-cholesterol, or both, in the blood of a mammal, by administering orally to said mammal food or pharmaceutical compositions containing an effective amount of a policosanol ester or mixture of policosanol esters wherein the acid moiety of the esters is a carboxilic acid containing from 2 to 22 carbon atoms.

The present invention may further provide food or pharmaceutical compositions for lowering LDL-cholesterol and triglycerides level or elevating HDL-cholesterol level, or both, in the blood of a mammal. Said compositions comprise an ester of a phytosterol and an omega-3 long chain polyunsaturated fatty acid such as eicosapentaenoic acid (EPA), docosahexaenoic acid (DHA), linolenic acid or an ester of a phytosterol and an omega-6 long chain polyunsaturated fatty acid such as linoleic acid or arachidonic acid, or a mixture of said esters.

The present invention enables a method for lowering LDL-cholesterol and triglycerides level or elevating HDL-cholesterol level, or both, in the blood of a mammal, by administering orally to said mammal food or pharmaceutical compositions containing an effective amount of an ester of a phytosterol, preferably beta-sitosterol or beta-sitostanol, and an omega-3 long chain polyunsaturated fatty acid such as cicosapentaenoic acid (EPA), docosahexaenoic acid (DHA), linolenic acid or an ester of a phytosterol and an omega-6 long chain polyunsaturated fatty acid such as linoleic acid or arachidonic acid, or a mixture of said esters.

The provision of food or pharmaceutical compositions for lowering LDL-cholesterol triglycerides level or raising HDL-cholesterol level, or both, in the blood of a mammal, may be achieved also by means of a composition comprising mixtures formed by one or more policosanol esters and one or more esters of phytosterols and an omega-3 long chain polyunsaturated fatty acid such as eicosapentaenoic acid (EPA), docosahexaenoic acid (DHA), linolenic acid or an ester of a phytosterol and an omega-6 long chain polyunsaturated fatty acid such as linoleic acid or arachidonic acid.

A method for lowering LDL-cholesterol and triglycerides level or raising HDL-cholesterol level, or both, in the blood of a mammal, is enabled by orally administering to said mammal food or pharmaceutical containing an effective amount of a mixture formed by one or more policosanol esters and one or more esters of a phytosterol, preferably beta-sitosterol or beta-sitostanol, and an omega-3 long chain polyunsaturated fatty acid such as eicosapentaenoic acid (EPA), docosahexaenoic acid (DHA), linolenic acid or an ester of a phytosterol and an omega-6 long chain polyunsaturated fatty acid such as linoleic acid or arachidonic acid.

### Description of the invention

Policosanol esters utilized in the present invention were prepared by transesterification of a mixture containing policosanols and a mixture containing ethyl or methyl esters of fatty acids using sodium ethylate as catalyst.

Policosanols from 20 to 26 carbon atoms can be obtained from the neutral fraction of tall oil as described in Chilean patent application N°873/98. Other sources such as sugarcane wax, rice bran wax are suitable to the purposes of this invention. Table I shows the average composition of policosanols in Tall Oil, Rice Bran Wax and Sugarcane Wax.

From Table I it is possible to observe that the three sources do not provide a complete range of 20 to 36 carbon atom policosanols separately, but they do together.

**Table I: Relative composition of fatty alcohols obtained from different sources**

| **Policosanol** | **Tall oil** | **Rice bran wax** | **Sugarcane wax** |
|---|---|---|---|
| Eicosanol C20 | 0,2 | -- | -- |
| Heneicosanol C21 | 0,1 | -- | -- |
| Docosanol C22 | 50,7 | 1,1 | -- |
| Tricosanol C23 | 2,7 | -- | -- |
| Tetracosanol C24 | 45,0 | 11,6 | 0,7 |
| Pentacosanol C25 | 0,3 | -- | -- |
| Hexacosanol C26 | 1,0 | 10,6 | 8,0 |
| Heptacosanol C27 | -- | -- | 3.5 |
| Octacosanol C28 | -- | 20,2 | 66,0 |
| Nonacosanol C29 | -- | -- | 0,8 |
| Triacontanol C30 | -- | 30,1 | 13,5 |
| Dotriacontanol C32 | -- | 16,8 | 6,0 |
| Tetratriacontanol C34 | -- | 8,0 | 1,5 |
| Hexatriacontanol C36 | -- | 1,4 | -- |

The ethyl or methyl esters of fatty acids of the present invention are obtained from vegetable or animal oils by methods well kown in the state of the art. These techniques comprise saponifying of oil followed by the separation of glycerol and soaps resulting from the saponifying process. Soaps are acidulated and then transformed into fatty acids and these fatty acids esterified with methanol or ethanol using sulfuric acid as catalyst.

The process of production of policosanol esters is carried out in a solvent free process, therefore this esters, that have good miscibility with fats and oils, can be safely incorporated into different fatty foods such as edible oil, margarine, mayonnaise, sauces, or milk. Thus, an objective of the present invention is achieved providing a food composition containing forms of policosanol easily absorbable in the digestive tract of a mammal, suitable for lowering LDL-cholesterol level or elevating HDL-cholesterol level in blood or both of said mammal. These easily absorbable forms of policosanol are the policosanol esters of the present invention, which incorporated into some suitable food substance such as table margarine, shortenings, ice cream, yogurt and others, form food compositions suitable for lowering LDL-cholesterol level or elevating HDL-cholesterol level in blood or both of a mammal, upon ingestion by said mammal an effective amount of the food composition.

Likewise, policosanol esters can be incorporated into pharmaceutical compositions in the form of capsules. These capsules may also comprise a pharmaceutically acceptable component such as an excipient, diluent, antioxidant, coloring agent and stabilizer. Pharmaceutical composition can also be provided in the form of tablets containing policosanol esters which may also comprise a pharmaceutically acceptable component, such as an excipient, coloring agent, antioxidant, binder and satabilizer. Said tablets and capsules form pharmaceutical compositions suitable for lowering LDL-cholesterol level or elevating HDL-cholesterol level in blood or both of a mammal, upon ingestion by said mammal an effective amount of the pharmaceutical composition.

A further objective which is to provide food or pharmaceutical compositions suitable for lowering LDL-cholesterol and triglycerides level or elevating HDL-cholesterol level in blood of a mammal or both, can be achieved esterifing a phytosterol with an omega-3 or omega-6 long chain polyunsaturated fatty and incorporating said esters into some suitable food substance such as table margarine, shortenings, ice cream, yogurt and others or in pharmaceutical forms such as tablets or capsules or both which may also comprise a pharmaceutically acceptable component such as an excipient, coloring agent, antioxidant, binder and stabilizer.

Still a further objective of the present invention enables a method for lowering LDL-cholesterol and triglycerides level or elevating HDL-cholesterol level in blood of a mammal or both, by administering orally to said mammal an effective amount of food or pharmaceutical composition comprising a phytosterol, preferably beta-sitosterol or beta-sitostanol, with an omega-3 or omega-6 long chain polyunsaturated fatty, said esters incorporated into some suitable food substance such as table margarine, shortenings, ice cream, yogurt and others or in a pharmaceutical forms such as tablets or capsules or both which may also comprise a pharmaceutically acceptable component such as an excipient, coloring agent, antioxidant, binder and stabilizer.

Food and pharmaceutical compositions suitable for lowering LDL-cholesterol and triglycerides level or elevating HDL-cholesterol level in blood of a mammal or both, can also be provided by incorporating one or more policosanol ester and one or more esters of a phytosterol and an omega-3 or omega-6 long chain polyunsaturated fatty acid into some suitable food substance such as table margarine, shortenings, ice cream, yogurt and othersor in pharmaceutical forms such as tablets or capsules or both which may also comprise a pharmaceutically acceptable component such as an excipient, coloring agent, antioxidant, binder and stabilizer.

A method of lowering LDL-cholesterol and triglycerides level or elevating HDL-cholesterol level in blood of a mammal or both, is enabled by administering orally to said mammal an effective amount of food or pharmaceutical composition comprising one or more policosanols esters and one or more esters of a phytosterol and an omega-3 or omega-6 long chain polyunsaturated fatty acid incorporated into some suitable food substance such as table margarine, shortenings, ice cream, yogurt and others or in in pharmaceutical forms such as tablets or capsules or both which may also comprise a pharmaceutically acceptable component such as an excipient, coloring agent, antioxidant, binder and stabilizer.

The following examples are presented in illustration of the compositions of this invention.

### Example 1. Preparation of policosanols esters.

104.3 g of ethyl-PUFA and 98.5 g of a mixture of policosanols were mixed in a 500-ml flask, the mixture were heated at the temperature of 180°C and the pressure of 5 mbar for 120 minutes to remove air from the mixture. After breaking the vacuum with nitrogen, 2.5 g o sodium ethylate were added to the flask and the mixture was further heated at the reduced pressure for 24 hours. After breaking the vacuum with nitrogen and removing the reaction mixture this was mixed with hot water to remove the catalists, the oily phase was separated and vacuum dried obtaining 103.1 g of policosanols esters.

### Example 2. Preparation of a food composition with policosanol ester.

A portion of policosanol esters from Example 1 was mixed with corn oil (3% in weight of the mixture) and a mayonnaise with the following composition was prepared:

| Ingredient | % |
|---|---|
| 3% oil-policosanol mixture | 70.0 |
| Thickening agent | 1.5 |
| Salt | 1.0 |
| Sugar | 1.0 |
| Vinegar (4 % in weight) | 6.0 |
| Water | 17.0 |
| Soy lecithin | 1.5 |
| Mustard | 2.0 |
| Total | 100.0 |

Mayonnaise was prepared using a home homogenizer. Its organoleptic properties did not differ from conventional mayonnaise.

### Example 3. Preparation of esters of phytosterol and PUFA

118 4 g of ethyl-PUFA .and 140.0 g of a mixture of phytosterols were mixed in a 500-ml flask, the mixture were heated at the temperature of 95 °C and the pressure of 5 mbar for 120 minutes to remove air from the mixture. After breaking the vacuum with nitrogen, 4.2 g o sodium ethylate were added to the flask and the mixture was further heated at the reduced presure for 24 hours. After breaking the vacuum with nitrogen and removing the reaction mixture this was mixed with hot water to remove the catalists, the oily phase was separated and vacuum dried obtaining 156.3 g of esters of phytosterol and PUFA.

### Example 4. Preparation of a food composition with esters of phytosterol and PUFA.

A portion of phytosteryl-PUFA from Example 3 was mixed with lard. 1000 g of lard were melted at 100°C in water bath and 10 g of esters of phytosterol and PUPA were incorporated. The lard was used for the elaboration of bread containing 20% of fatty matter with respect to flour used. The organoleptic characteristics of bread do not differ from conventional bread.

### Example 5. Short term nutritional evalution in rats. Effect of esters of phytosterol and PUFA on serum and hepatic lipids in rats

24 Sprague Dawley male rats divided into four groups of six animals each were fed for nine days with the following diet: the C0 group was fed with a basal food comprising *Champion* pellets ground and powdered and mixed with corn oil (3.3% in weight of the mixture). The Cl group was fed with mixture comprising basal and cholesterol (1% in weight of the mixture). The A1 group was fed with a mixture comprising basal food, 1% of cholesterol and 1% of stanol esters in weight of the mixture. Finally, the A2 group was fed with a mixture comprising the basal food, 1% of cholesterol and 1% of esters of phytosterol and PUFA in weight of the mixture.

The stanol esters comprised a mixture of beta-sitostanol and campestanol esters of fatty acids obtained from rape seed oil. Esters of phytosterol and PUFA esters were prepared according to Example 3. Dietary treatment was individually applied and corporal weight and dietary ingesta were measured. After the nine days of feeding, total lipids and cholesterol in the liver and cholesterol and triglycerides in the blood serum of each animal were determined. Tables I and II show the results:

**Table I: Total lipids and total cholesterol in the liver**

| | Total lipids | | Cholesterol | |
|---|---|---|---|---|
| | (mg/g liver) | | (mg/g liver) | |
| C₀ | 34.99±2.23 | (5) | 1.53±0.12 | (5) |
| C₁ | 40.22±0.99 | (5) | 2.82±0.19 | (6) |
| A₁ | 30.66±1.44 | (6) | 1.28±0.15 | (5) |
| A₂ | 28.79±1.48 | (4) | 0.99±0.004 | (5) |

Figures represent mg/g of liver and the results are presented as an average per group±standard error of the sample. The number of samples analyzed appears in parentheses.

Pairwise comparison of the means using Student test, indicate that there is a significant difference between C1 and A1 or A2 in total lipids and total cholesterol at a significance level of 5% in both cases. Also, there is a significant difference between A1 and A2 in total lipids and total cholesterol at 10% and 5% level of significance respectively.

**Table II: Total cholesterol and triglycerides in blood serum.**

| | Total cholesterol | Triglycerides |
|---|---|---|
| C0 | 68.44 ±7.13 (6) | 21.75 ± 2.16 (6) |
| C1 | 140.17 ± 7.80 (6) | 34.11 ± 3.36 (5) |
| A1 | 120.68 ± 11.14 (5) | 33.42 ± 6.26 (4) |
| A2 | 126.10 ± 3.81 (5) | 29.74 ± 4:13 (5) |

Figures represent mg/dl and the results are presented as an average per group±standard error of the sample. The number of analyzed samples appears in parentheses.

From the results it is possible to conclude that a significant difference exists between Cl and A1 or A2 in total serum cholesterol at a significance level of 1%, but the difference between A1 and A2, is not significant at a significance level of 10%. Concerning triglycerides, there is no significant difference with a significance level of 10% between C1 and A1, but between the difference between C1 and A2 is significant at a significance level of 10%. Likewise, between A1 and A2, there is a significant difference with a significance level of 20%.

Blood serum level of HDL-cholesterol in A1 and A2 were also measured and results are shown in Table III.

**Table III: Serum level of HDL cholesterol**

| | HDL |
|---|---|
| A1 | 37.96 ± 1.97 (6) |
| A2 | 41.78 ± 1.65 (5) |

Figures represent mg/dl and results are presented as an average per group±standard error of the sample. The number of analyzed samples appears in parentheses.

HDL-cholesterol is higher in A2 group than in the A1 group with a significance level of 1%.

## Claims

1. A composition for lowering LDL-cholesterol or elevating HDL-cholesterol in blood of a mammal, **characterised in that** the composition comprises an ester of a carboxylic acid with eicosanol, heneicosanol, tricosanol, docosanol, tetracosanol, pentacosanol or hexacosanol.

2. A composition according to claim 1, **characterised in that** it further comprises a food substance,

3. A composition according to claim 2, **characterised in that** the food substance is selected from the group consisting of table margarine, shortening, mayonnaise, vegetable oil, ice cream, milk and yoghurt.

4. A composition according to claim 1, **characterised in that** it further comprises a pharmaceutically acceptable component selected from the group consisting of an excipient, diluent, antioxidant, coloring agent, binder and stabilizer.

5. A composition for lowering LDL-cholesterol or elevating HDL-cholesterol in blood of a mammal or both, **characterised in that** it comprises an ester of a policosanol or a mixture of eaters of policosanols and an ester of a phytosterol or a mixture of esters of phytosterols.

6. A composition according to claim 5, **characterised in that** the acid moiety of the ester of policosanol or the mixture of esters of policosanols is a carboxylic acid containing from 2 to 22 carbon atoms.

7. A composition according to claim 5 or 6, **characterised in that** the acid moiety of the ester of phytosterol or the mixture, of esters of phytosterols is a fatty acid selected from the group consisting of eicosapentaenoic acid, docosahexaenoic acid, linoleic acid, linolenic acid and arachidonic acid.

8. A composition according to claim 7, **characterised in that** it further comprises a food substance or a mixture of food substances.

9. A composition according to claim 8, **characterised in that** the food substance or the mixture of food substances is selected from the group consisting of table margarine, shortening, mayonnaise, vegetable oil, ice cream, milk and yoghurt.

10. A composition according to claim 5, **characterised in that** it further comprises a pharmaceutically acceptable component selected from the group consisting of an excipient, diluent, antioxidant, coloring agent, binder and stabilizer.

## Patentansprüche

1. Zusammensetzung zum Senken des LDL-Cholesterins oder Erhöhen des HDL-Cholesterins im Blut eines Säugetiers, **dadurch gekennzeichnet, dass** die Zusammensetzung einen Ester einer Karboxylsäure mit Alcosanol, Heneicosanol, Tricosanol, Docosanol, Tetracosanol, Pentacosanol oder Hexacosanol umfasst.

2. Zusammensetzung nach Anspruch 1, **dadurch gekennzeichnet, dass** sie ferner eine Nahrungsmittelsubstanz umfasst.

3. Zusammensetzung nach Anspruch 2, **dadurch gekennzeichnet, dass** die Nahrungsmittelsubstanz aus der aus Speisemargarine, Backfett, Majonäse, Pflanzenöl, Eiscreme, Milch und Joghurt bestehenden Gruppe gewählt wird.

4. Zusammensetzung nach Anspruch 1, **dadurch gekennzeichnet, dass** sie ferner einen pharmazeutisch annehmbaren Bestandteil umfasst, welcher aus der aus einem Arzneistoffträger, Verdünnungsmittel, Antioxidans, Farbstoff, Bindemittel und Stabilisator bestehenden Gruppe gewählt wird.

5. Zusammensetzung zum Senken des LDL-Cholesterins oder Erhöhen des HDL-Cholesterins im Blut eines Säugetiers oder beides, **dadurch gekennzeichnet, dass** sie einen Ester eines Policosanols oder eine Mischung von Estern von Policosanolen und einen Ester von Phytosterol oder eine Mischung von Estern von Phytosterolen enthält.

6. Zusammensetzung nach Anspruch 5, **dadurch gekennzeichnet, dass** der Säureanteil des Esters von Polycosanol oder der Mischung von Estern von Policosanolen eine Karboxylsäure ist, welche von 2 bis 22 Kohlenstoffatome enthält.

7. Zusammensetzung nach Anspruch 5 oder 6, **dadurch gekennzeichnet, dass** der Säureanteil des Esters von Phytosterol oder der Mischung von Estern von Phytosterolen eine Fettsäure ist, die aus der aus Eicosapentaenoinsäure, Docosahexaenoinsäure, Linoleinsäure, Linoleninsäure und Arachidoninsäure bestehenden Gruppe gewählt ist.

8. Zusammensetzung nach Anspruch 7, **dadurch gekennzeichnet, dass** sie ferner eine Nahrungsmittelsubstanz oder eine Mischung von Nahrungsmittelsubstanzen enthält.

9. Zusammensetzung nach Anspruch 8, **dadurch gekennzeichnet, dass** die Nahrungsmittelsubstanz oder die Mischung von Nahrungsmittelsubstanzen aus der aus Speisemargarine, Backfett, Majonäse, Pflanzenöl, Eiscreme, Milch und Joghurt bestehenden Gruppe gewählt ist.

10. Zusammensetzung nach Anspruch 5, **dadurch gekennzeichnet, dass** sie ferner einen pharmazeutisch annehmbaren Bestandteil enthält, welcher aus der aus einem Arzneistoffträger, einem Verdünnungsmittel, einem Antioxidans, einem Farbstoff, einem Bindemittel und einem Stabilisator bestehenden Gruppe gewählt ist.

## Revendications

1. Composition pour abaisser le taux de cholestérol-LDL ou élever le taux de cholestérol-HDL dans le sang d'un mammifère, **caractérisée en ce qu'**elle comprend un ester d'un acide carboxylique avec l'eicosanol, l'henéicosanol, le tricosanol, le docosanol, le tétracosanol, le pentacosanol ou l'hexacosanol.

2. Composition suivant la revendication 1, **caractérisée en ce qu'**elle comprend en outre une substance alimentaire.

3. Composition suivant la revendication 2, **caractérisée en ce que** la substance alimentaire est choisie dans le groupe consistant en la margarine de table, une matière grasse, la mayonnaise, une huile végétale, une crème glacée, le lait et le yaourt.

4. Composition suivant la revendication 1, **caractérisée en ce qu'**elle comprend en outre un constituant pharmaceutiquement acceptable choisi dans le groupe consistant en un excipient, un diluant, un antioxydant, un colorant, un liant et un stabilisant.

5. Composition pour abaisser le taux de cholestérol-LDL ou élever le taux de cholestérol-HDL, ou bien pour parvenir à ces deux actions, dans le sang d'un mammifère, **caractérisée en ce qu'**elle comprend un ester d'un policosanol ou un mélange d'esters de policosanols et un ester d'un phytostérol ou un mélange d'esters de phytostérols.

6. Composition suivant la revendication 5, **caractérisée en ce que** le groupement acide de l'ester de policosanol ou du mélange d'esters de policosanols est un acide carboxylique contenant 2 à 22 atomes de carbone.

7. Composition suivant la revendication 5 ou 6, **caractérisée en ce que** le groupement acide de l'ester de phystérol ou du mélange d'esters de phystérols est un acide gras choisi dans le groupe consistant en l'acide eicosapentaénoïque, l'acide docosahexaénoïque, l'acide linoléïque, l'acide linolénique et l'acide arachidonique.

8. Composition suivant la revendication 7, **caractérisée en ce qu'**elle comprend en outre une substance alimentaire ou un mélange de substances alimentaires.

9. Composition suivant la revendication 8, **caractérisée en ce que** la substance alimentaire ou le mélange de substances alimentaires est choisi dans le groupe consistant en la margarine de table, une matière grasse, la mayonnaise, une huile végétale, une crème glacée, le lait et le yaourt.

10. Composition suivant la revendication 5, **caractérisée en ce qu'**elle comprend en outre un constituant pharmaceutiquement acceptable choisi dans le groupe consistant en un excipient, un diluant, un antioxydant, un colorant, un liant et un stabilisant.
